# EUROPEAN PATENT SPECIFICATION

(11) **EP 2 792 749 B1**
(45) Date of publication and mention of the grant of the patent: **22.11.2017**
(21) Application number: 12851604.4
(22) Date of filing: 23.11.2012
(51) Int. Cl.: C12N 15/70, C12N 1/20, C12N 1/21, C12N 15/67, C07K 1/18, C12R 1/19, C07K 14/245, C07K 14/00, C07K 14/31, C12P 21/02, C12N 15/62

(54) **METHOD FOR HIGHLY EXPRESSING RECOMBINANT PROTEIN OF ENGINEERING BACTERIA AND USE THEREOF**
VERFAHREN ZUR STARKEN EXPRESSION REKOMBINANTER PROTEINE AUS MANIPULIERTEN BAKTERIEN UND IHRE VERWENDUNG
PROCÉDÉ POUR L'EXPRESSION MASSIVE D'UNE PROTÉINE RECOMBINANTE DE BACTÉRIES GÉNÉTIQUEMENT MODIFIÉES ET UTILISATION ASSOCIÉE

(30) Priority: 25.11.2011 CN 201110380864
(43) Date of publication of application: 22.10.2014
(73) Proprietor: Protein Design Lab, Ltd., Beijing 100095 (CN)
(72) Inventor: QIU, Xiaoqing, Beijing 100095 (CN)
(74) Representative: Grünecker Patent- und Rechtsanwälte PartG mbB
(86) International application number: PCT/CN2012/085182
(87) International publication number: WO 2013/075660

(56) References cited:
- EP-A1- 2 474 558
- WO-A1-2011/026447
- CN-A- 100 999 553
- CN-A- 101 633 699
- CN-A- 101 643 501
- CN-A- 102 653 779
- US-A1- 2004 058 415
- AIDAN J. DOHERTY ET AL: "A superior host strain for the over-expression of cloned genes using the T7 promoter based vectors", NUCLEIC ACIDS RESEARCH, vol. 23, no. 11, 1 January 1995 (1995-01-01), pages 2074-2075, XP055194412, ISSN: 0305-1048, DOI: 10.1093/nar/23.11.2074
- ZINDER N D ET AL: "The filamentous phage (Ff) as vectors for recombinant DNA- a review", GENE, ELSEVIER, AMSTERDAM, NL, vol. 19, no. 1, 1 July 1982 (1982-07-01), pages 1-10, XP023502394, ISSN: 0378-1119, DOI: 10.1016/0378-1119(82)90183-4 [retrieved on 1982-07-01]
- QIU X-Q ET AL: "An engineered multidomain bactericidal peptide as a model for targeted antibiotics against specific bacteria", NATURE BIOTECHNOLOGY, NATURE PUBLISHING GROUP, US, vol. 21, no. 12, 1 December 2003 (2003-12-01), pages 1480-1485, XP003010122, ISSN: 1087-0156, DOI: 10.1038/NBT913
- YAO, WENBING ET AL.: 'The study on culture of engineering strain and optimization of fermentation conditions of recombinant human interferon alpha-2b' JOURNAL OF CHINA PHARMACEUTICAL UNIVERSITY vol. 31, no. 6, 30 December 2000, pages 462 - 465, XP008173175

## Description

### BACKGROUND OF INVENTION

In earlier studies, the inventor conducted creative experiments and identified a series of new recombinant peptides with Colicin as attack point, which operationally connects a polypeptide (natural or artificial design) with identification and binding ability to target cells. For example, the new antibiotic PMC-AM1 disclosed in patent No. ZL200910092128.4, (publication number CN101633699), named "Novel antibiotic comprising an antibody mimetic, its preparation and uses thereof', shows a broad-spectrum antibiotic property and has stronger antibacterial activity on *Neisseria meningitidis, Multidrug-resistance Pseudomonas aeruginosa, Vancomycin-resistant Enterococcus faecalis* or *Methicillin-resistant Staphylococcus aureu* compared to the known antibiotics. The inventor's further invention entitled "A novel antibiotic, its nucleotide sequence, methods of construction and uses thereof", with CN patent No.ZL200910157564.5 (publication number CN101643501), disclosed a series of new anti-staphylococcus antibiotics, such as PMC-SA1, PMC-SA2, PMC-SA3, PMC-SA4, PMC-SE as well as PMC-PA. In in vivo and in vitro experiments, these antibiotics showed better targeting ability and stronger antibacterial activity than current antibiotics, antifungal antibiotic and chemotherapeutic drugs. Additionally compared with current antibiotics, these new antibiotics showed incomparable biological security and anti drug-resistance characteristic.

The foresaid novel antibiotics as a whole are a kind of water-soluble proteins with 600 amino acid residues, but in which there is a hydrophobic domain with 40 amino acid residues near the carboxy-terminus. Compared to the preparation of other water-soluble proteins with single structure, it is more difficult to assemble and express the novel antibiotics, which inevitably affects protein yield. It is necessary to improve current expression processes to achieve high yield and purity of the novel antibiotics. It will make sense for bringing the novel antibiotics into actual clinical application and practice.

### SUMMARY OF THE INVENTION

According to the peptide structure and characteristics of the new antibiotics disclosed in the current patent application, the present invention provides a method for highly expressing recombinant protein from engineered bacteria.

In one aspect, the present invention provides a method for highly expressing recombinant protein from engineered bacteria, wherein the end with hydrophilicity of the recombinant protein is colicin polypeptide and the other end with hydrophobic nature is polypeptide of target moiety which is capable of binding target, the method comprising:
(1) transfecting recombinant plasmid for the expression of the recombinant protein into E. coli engineered bacteria with pET system to obtain positive monoclonal colonies,
(2) producing seed bacteria solution of the positive monoclonal colonies, and inducing protein expression and large-scale culturing of the seed bacteria solution; the supernatant of the large-scale cultured solution contains the expressed recombinant protein,
(3) extracting and purifying the recombinant protein from the supernatant, wherein the *E. coli* engineered bacteria with pET system is E.coli B834 (DE3), wherein the large-scale culturing medium used for said large-scale culturing of the seed bacteria solution has water as solvent and comprises the following components: NaCl 6.0∼6.7g/L, peptone 25.0g/L, yeast powder 7.5g/L, glucose 0.6∼2.0g/L, Na₂HPO₄·7H₂O 6.8∼8.3g/L, KH₂PO₄ 3.0∼4.3g/L, NH₄Cl 1.0∼1.4g/L, MgSO₄ 0.2∼0.4g/L, CaCl₂0.01g/L, methionine 0∼40mg/L.

In a preferable exemplary embodiment, said large-scale culturing medium has water as solvent and comprises following components: NaCl 6.0g/L, peptone 25.0g/L, yeast powder 7.5g/L, glucose 2.0g/L, Na₂HPO₄·7H₂O 6.8g/L, KH₂PO₄ 3.0g/L, NH₄Cl 1.0g/L, MgS0₄ 0.2g/L, CaCl₂0.01g/L, methionine 0∼40mg/L.

In some exemplary embodiments, said large-scale culturing of the seed bacteria solution comprises following steps: the seed bacteria liquid was added into a container and started growth for 2 to 3 hours at 30°C, when the OD value reached 0.4-0.6, the seed bacteria solution was conducted heat shock at 42°C for 30 minutes, and then cooled down to 37°C and kept growing for 1.5 to 2 hours before being collected.

In some exemplary embodiments, wherein in conducting heat shocks the IPTG with the final density 0.5mmol/L was added into said large-scale culturing medium.

In some exemplary embodiments, said extraction and purification of the recombinant protein from the supernatant is performed by CM ion exchange column, and the loading quantity of the supernatant depends on the ratio value which is 2.5mg/ml between the weight of the recombinant protein in the supernatant and the volume of Gel particles used in the CM ion exchange column. In some exemplary embodiments, the elution buffer used for said extraction and purification by CM ion exchange column is boric acid buffer solution with 0.2mo1/L NaCl.

In most exemplary embodiments, said recombinant plasmid for the expression of the recombinant protein is selected from the group consisting of pBHC-SA1, pBHC-SA2, pBHC-SA3, pBHC-SA4, pBHC-SE, pBHC-PA and pBHC-PorA1.

In a further aspect, the present invention provides the use of any of the above methods for preparing the recombinant peptides PMC-SA1, PMC-SA2, PMC-SA3, PMC-SA4, PMC-SE, PMC-PA or PMC-AM.

In another aspect, the present invention provides a medium used for *E. coli* engineered bacteria with pET system, the medium has water as solvent and comprises following components: NaCl 6.0∼6.7g/L, peptone 25.0g/L, yeast powder 7.5g/L, glucose 0.6∼2.0g/L, Na₂HPO₄·7H₂O 6.8∼18.3g/L, KH₂PO₄ 3.0∼4.3g/L, NH₄Cl 1.0∼1.4g/L, MgSO₄ 0.2∼0.4g/L, CaCl₂0.01g/L, methionine 0∼40mg/L.

In some exemplary embodiments, said *E. coli* engineered bacteria with pET system is *E. coli* B834 (DE3), and the medium has water as solvent and comprises following components: NaCl 6.0g/L, peptone 25.0g/L, yeast powder 7.5g/L, glucose 2.0g/L, Na₂HPO₄·7H₂O6.8g/L, KH₂PO₄ 3.0g/L, NH₄Cl1.0g/L, MgSO₄0.2g/L, CaCl₂0.01g/L, methionine 0∼40mg/L.

The pET expression system provided by Novagen Company is a common system for cloning and expressing recombinant proteins in *Escherichia* coli. In this invention, a series of BL-21 (DE3) cells is transfected with recombinant mutated plasmid disclosed in former patents resulting in a higher protein expression yield compared to the TG1 cells of this invention. By experimental data, we found that B834 (DE3), which is the parent strain of BL-21 (DE3), has a more ideal expression productivity than BL-21 (DE3). The experimental data showed that the B834 (DE3) has significantly increased protein expression levels compared to the TG1 system.

Medium is used for providing required carbon source, nitrogen source and inorganic salts for bacterium growth and multiplication. According to the present invention, a medium with capability of improving the expression level of a target protein is provided, which has a optimum composition for engineered bacteria fermentation. In this invention, the medium, named FB-M9 compound medium has an increased carbon source, nitrogen source and MgSO4, CaCl2 as well as some special amino acids that are required for growth of engineered bacteria with pET system. The medium moderately improved engineered bacteria reproduction speed and protein expression rate. Furthermore, material cost of the improved medium is relatively low, which provides larger research space and higher development value for large-scale production in the future.

According to the product manual guide, the loading capacity of CM ion gel particles used in purification system in this invention could not reach the ideal standard described in the product manual, which limits the recovery rate of target protein. In the present invention, the recovery rate has been significantly improved by the means of reducing loading quantity of sample while moderately increasing the gel volume, etc. The result also reflected that it is necessary to find or develop a kind of ion exchange gel which is more efficient for large-scale industrial production of the target protein. In addition, the recombinant protein has fewer impurities owing to elution with optimized concentration used in the ion exchange steps of this invention.

In summary, this invention provides a variety of optional improved methods of protein expression from engineered E. coli bacteria by the means of choosing engineered strains, optimizing the composition of medium, improving the purification and recovery rate, etc. This also provides a possible research direction and technical route for finally finding optimal procedure for highly efficient expression of fusion protein needed. Compared to the original expression system disclosed in former patents, the expression system developed in the present invention has significantly improved the expression level of fusion protein, and provided a beneficial basis of theory and practice for the subsequent large-scale industrial production.

### BRIEF DESCRIPTION OF THE DRAWINGS

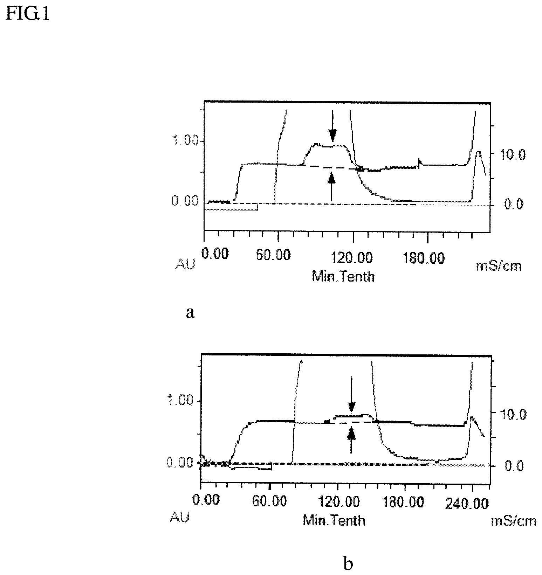

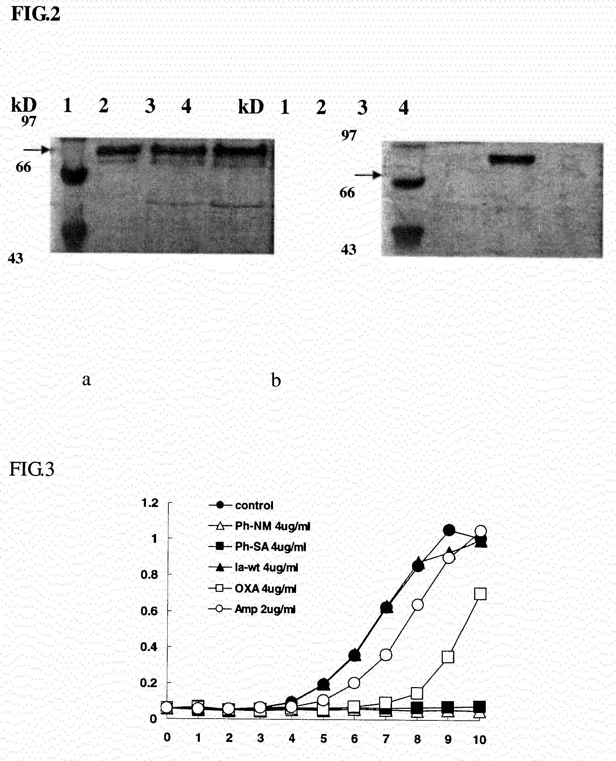

Fig. 1 shows the conductance value of eluent in the protein elution process with different volume gel column.
   a. The elution process of protein with 150ml CM gel column.
   b. The elution process of protein with 600ml CM gel column.
      The curve indicated by two arrows in the figure represents the conductance value of the eluent. The area indicated by the arrows is a conductance peak caused by the loss of the sample PMC-SA in loading process. The area of the conductance peak caused by the loss of the sample PMC-SA is reduced by 70% after increasing the volume of gel.
      Another curve: OD value of elutropic protein
Fig. 2 shows SDS-PAGE Gel electrophoresis of the PMC-S
   From left to right in the order:
   a. 1. Marker, 2. PMC-SA1 produced by TG1, 3. PMC-SA1 produced by BL-21, 4. PMC-SA1 produced by B834;
   b. 1. Marker, 2. PMC-SA1 eluted by boric acid buffer solution with 0.1 M NaCl, 3. PMC-SA1 eluted by boric acid buffer solution with 0.2 M NaCl, 4. PMC-SA1 eluted by boric acid buffer solution with 0.3 M NaCl.
Fig. 3. Shows the inhibition curve of the PMC-SA against MRSA (BAA42)
   Y-axis represents light absorption value; X-axis represents bacterial growth time
   Control: control group; Amp: ampicillin sodium; OXA: oxacillin; Ia-wt: wild type colicin Ia; PMC-SA1: anti-staphylococcus aureus polypeptide;
   PMC-AM: anti-diplococcus meningitides polypeptide.

### DETAILED DESCRIPTION OF THE INVENTION

Following examples are used for explaining the invention.

The experimental equipment and instruments used in the present invention are as follows:

### 1. Bacterial strain

E. coli TG1 engineered bacteria (AECOM, K. Jakes).

E. coli BL-21(DE3), B834(DE3), Nova Blue(DE3) and 618 engineered bacteria are all purchased from Novagen company.

Staphylococcus aureus ATCC BAA-42 is purchased from ATCC (American Type Culture Collection).

Plasmid: pBHC-SA1,pBHC-SA2,pBHC-SA3, pBHC-SA4, pBHC-SE , pBHC-PA, pBHC-PorA1 (These plasmids are recorded in patents ZL200910092128.4 (publication number CN101633699) and ZL 200910157564.5 (publication number CN101643501), and preserved in the inventor's laboratory. The inventor promised to offer them to the public for necessary verification tests)

### 2. Main reagents and medicine

Yeast powder (OXIOD LP0021), peptone (OXIOD LP0042) as well as other chemical reagents are all analytical reagents;
Dialysis bag Snake Skin Dialysis Tubing (Pierce, intercept molecular weightl×104, Lot#KD32324);
Streptomycin Sulfate for injection (NCPC)
AMP ampicillin sodium for injection (Harbin pharmaceutical)
Anion exchange column gel (Pharmacia Biotech CM Sepharose Fast Flow LotNo.225016).
LB liquid medium: Sodium chloride 1g, peptone 1g, and yeast 0.5g were added into a 250 ml flask with the addition of 100 ml water, dissolved and autoclaved at 120°C for 8min.

LB solid medium: 100ml LB solid medium containing sodium chloride 0.5-1.5g, peptone 0.5-2g, yeast 0.3-1g and agar 0.8-3g. The LB solid medium is used for plate culture of single colony after strain recovery. Reagents were added into a 250 ml flask with the addition of 100 ml water, dissolved and autoclaved at 120°C for 8min.

FB-M9 complex medium: NaCl 6.0∼6.7g/L, peptone 25.0g/L, yeast powder 7.5g/L, glucose 0.6∼2.0g/L, Na₂HPO₄·7H2O 6.8∼18.3g/L, KH₂PO₄ 3.0∼ 4.3g/L, NH₄C1 1.0∼1.4g/L, MgSO₄ 0.2∼0.4g/L, CaCl₂0.01g/L, methionine 0∼40mg/L.

Improved FB-M9 complex medium: NaCl 6.0g/L, peptone 25.0g/L, yeast powder 7.5g/L, glucose 2.0g/L, Na₂HPO₄·7H₂O 6.8g/L, KH₂PO₄3.0g/L, NH₄Cl 1.0g/L, MgSO₄0.2g/L, CaCl₂0.01g/L, methionine 0∼40mg/L. The methionine is 40mg/L in the process with E.coli B834(DE3) as engineered bacteria.

### 3. Key Instrument

Bio-Rad Protein chromatography purification system (BioLogic Duo Flow, BioLogic Maximizer, BioLogic QuadTec UV-Vis Detector, BioLogic Econo Pump);
Ultrasonic Cell Disruptor (Soniprep 150), protein purification ion exchange column with 5cm diameter (Pharmacia Biotech XK50), protein purification ion exchange column with 11cm diameter (Shanghai Huamei);
Centrifuge (Beckman Coulter Avanti J-20XP, Beckman Coulter Avanti J-25);
Spectrophotometer (Bio-Rad Smart Spect Plus spectrophotometer);
Automatic fermenter (Bioengineering AG LP351-42L);

High pressure homogenizer (Italian NiroSoavi NS1001L2KSN 6564).

Statement: the biological materials adopted in this invention have been known before the application's filing date and have been also preserved in this applicant's lab. The applicant promised to offer them to the public for necessary verification tests in the twenty years since application's filing date.

### Example 1. The option experiment of engineered bacterial strains

Classic plasmid carrying the colicin Ia and its immune protein gene (GenBank M13819) are from laboratory of Dr. Finkelstein. (Qiu XQ et al. An engineered multi domain bactericidal peptide as a model for targeted antibiotics against specific bacteria. Nat Biotechnol, 2003; 21(12): 1480-1485). The classic plasmids were modified into the following seven kinds of restructuring mutation plasmids from former research: pBHC-SA1, pBHC-SA2, pBHC-SA3 pBHC-SA4, pBHC-SE, pBHC-PA, pBHC-PorA1.

### Step 1. Transformation of competent cell

40uL Novagen pET system engineered bacteria BL-21(DE3), B834(DE3), Nova Blue(DE3), 618 were respectively transformed with 100ng recombinant mutant plasmids pBHC-SA1, and then ice-incubated for 5 minutes, heat-shocked at 42°C for 30 seconds, kept in ice for 2 minutes, added with 160µl SOC medium and shake-cultivated at 220 rpm, 37°C for 1 hour and then coated (LB medium with 1 % agar and 50ug/ml ampicillin, and cultured overnight at 37°C). Single colonies are picked out and cultivated to obtain the seed strain, which is conserved at a low temperature.

### Step2: Strain Recovery

### 1. Preparing recovered bacteria solution

The conserved strain was thawed at 4°C; 1.5ml of the strain is transferred into 10ml LB medium (containing 50ug/ml of AMP) and cultivated at 220 rpm, 37 °C for 5-8 hours.

### 2. Inoculation of single colony

The recovered bacteria solution was diluted 10⁴ or 10⁵ times; and 10ul of the diluted bacteria solution was transferred on to LB solid medium plate (containing 50µg/ml of AMP) and coated on the plate. The plate was placed in a humid box and cultivated in incubator at 37°C for 10-12 hours until round single colonies have grown out on the surface of the medium.

### Step 3. Large-scale culturing of the single colonies (1)

Single colonies with regular round shape and smooth edge were picked up from the plate and respectively added into 1.5ml LB medium, and cultivated at 220rpm, 37 °C for 5-8hours.
(2) Each 1.5ml LB bacteria solution was transferred into a 100ml LB medium, and cultivated at 220rpm, 37 °C for 5-8hours.
(3) Primary stage of large-scale culturing: the 100ml of bacteria solution from the last step was added into 700ml of improved FB-M9 complex medium and cultivated at 220 rpm, 37 °C for 5-8 hours.
(4) Secondary stage of large-scale culturing: 700ml of bacteria solution from the primary stage is added into 6×700ml of the improved FB-M9 complex medium and cultivated at 220rpm, 37°C for 5-8hours.
(5) Third stage of large-scale culturing: 6×700ml of bacteria solution from the secondary stage was added into 20L of the improved FB-M9 complex medium and cultivated in a fermenter with stirring rate of 220rpm and maximum oxygen flow volume, 37°C for 3-5 hours.
(6) Fermentation of engineered bacteria and induced expression of protein: 20L of bacteria solution from the third stage of large-scale culturing was added into 200L of improved FB-M9 complex medium and cultivated in a fermenter for induced expression of protein with stirring rate of 220rpm and maximum oxygen flow volume, at 30°C for 2-4 hours; 42°C for 0.5 hours; and 37°C for 1∼2 hours, note that IPTG is added at 42°C with a final concentration of 0.5mM.

### Step 4. Collecting bacteria by centrifugation

6000 g fermentation liquor obtained from step 3 was centrifuged at 4°C for 20min. The precipitate was collected and added into 50mM boric acid buffer (pH9.0) for resuspension of the bacteria. Note : the boric acid buffer has 2mM PMSF (Phenylmethylsulfonyl fluoride serine protease inhibitor). All subsequent steps after bacteria resuspension was conducted at 4°C.

### Step5. Cells fragmentation

After complete suspension in pH9.0 boric acid buffer, the bacterial cells were fragmented by a High Pressure Homogenizer at 500-600 bar for 7 times, with intervals of 3-5 minutes.

### Step6. Precipitation of the bacterial DNA

The fragmented bacteria solution was centrifuged at 55000 g, 4°C for 40 min. The supernatant was supplemented with streptomycin sulfate (16 bottles of 1 million unit streptomycin sulfate were added into every 200ml liquid supernatant), and stirred for 1 h with a magnetic stirrer.

### Step7. Dialysis

The bacteria solution from step 6 was centrifuged at 55000g, 4°C for 20min. The supernatant was placed into a dialysis bag and dialyzed for 8∼12 hours in boric acid buffer, which was changed once every 4 hours.

### Step8. Purifying the protein medicine and obtaining antibacterial-engineered polypeptide

The dialyzed bacteria solution was centrifuged at 55000g, 4°C for 20min. The the protein concentration in the supernatant was measured in unit volume and placed into a Bunsen beaker for conducting protein purification by ion exchange method. The supernatant with known protein concentration was uploaded onto a CM ion exchange column. The sample loading and its ratio with the CM iron gel particle are in accordance with the Product Manuals of CM ion exchange column. After being washed completely the CM ion exchange column was eluted with 50mM boric acid buffer containing 0.3M NaCl to obtain the novel antibacterial-engineered polypeptide.

The results are shown in table 1, the expression efficiency of PMC-SA by ***E.coli*** B834 (DE3) is the highest.

**Table 1. Expression efficiency of different bacterial strains**

| (Average unit production=Gross production of extracted PMC-SA1/ volume of bacterial liquid) | | | | | |
|---|---|---|---|---|---|
| Engineered strain | TG1 | BL-21 | 618 | NavaBlue | B834 |
| Average unit production (mg/L) | 0.8 | 10 | 5.8 | 8.1 | 24.4 |

The same operation was conducted on the other six restructuring mutation plasmids, the results show a similar trend compared to the result listed in Table 1, namely, in contrast to other engineered bacteria, *E. coli* B834 (DE3) showed the highest expression efficiency on all seven restructuring mutation plasmids.

The operation of heat shock performed in order to induce protein expression in this embodiment was different from that in the prior art: After transferring the seed bacteria liquid into the tank, bacteria were cultured at an initial temperature 30°C for 2 hours, when OD value had reached 0.4-0.6, heat shock was conducted at 42 °C for 30 minutes, then when the temperature cooled down to 37 °C, the bacteria were cultured again for 1.5 to 2 hours. At this stage the OD value of bacteria liquid can reach 1-3 or even more, and the bacteria can be collected. During this process, 0.5mM IPTG was added to induce expression of pET engineered bacteria.

Before performing the present method, the usual process for preparing the recombinant peptides was as following:
100ng of the mutant plasmids was ice-incubated with 40ul competent cell of BL-21 engineered bacteria for 5 minutes, heat-shocked at 42°C for 30 seconds, ice-incubated for 2 minutes, added with 160ul of SOC medium, shake-cultivated at 220 rpm, 37°C for 1hour and then coated on plate (LB medium with 1% agar and 50ug/ml ampicillin, and cultured overnight at 37°C). Single colonies were picked out for large-scale culturing.

Large-scale culturing: 8-10L FB medium, 250rpm, at 37°C for 3-4 hours; was added with IPTG, 250rpm, at 28°C grown for 4 hours again; centrifuged to precipitate bacteria at 4°C, 6000g, 20 minutes. The precipitated bacteria was added with 80-100ml 50mM boric acid buffer (pH9.0, 2mM EDTA) kept at 4°C to suspend, then added with 50ug PMSF and broken by ultra sonication (4°C, 400 w, 1 minute, repeat 4 to 5 times with interrupted for 2-3 minutes for keeping the temperature of the liquid). Then, the broken bacteria was centrifuged at high speed (4°C, 75000g, 90minutes), the supernatant was supplemented with 5 million units streptomycin sulfate to precipitate DNA (4°C stirred for 1 hour), and centrifuged at 10000g, 4°C, for 10 minutes. The supernatant was transferred into a dialysis bag with the molecular weight 15000 on 4°C, and dialyzed by 10L 50 mM boric acid buffer overnight, then centrifuged at 4°C, 10000g, for 10 minutes once again. The supernatant was loaded on CM ion exchange column, and after complete washing and elution by 0.3M NaCl + 50mM boric acid buffer, the new antibiotics were obtained.

### Example 2: Improving medium

The classic FB medium for colicin Ia preparation (Qiu XQ et al. An engineered multi domain bactericidal peptide as a model for targeted antibiotics against specific bacteria. Nat Biotechnol, 2003 ; 21(12) : 1480-1485 /Karen Jakes, Charles Abrams, Alan Finkelstein, et al. Alteration of the pH-dependent Ion Selectivity of the Colicin E1 Channel by Site-directed Mutagenesis.JBC, 1990; 265(12): 6984-6991) has components as follows: peptone 25.0g/L, yeast powder 7.5g/L, NaCl 6.0g/L and glucose 1.0g/L.

In this invention, we used FB medium without glucose, comprising the following components: peptone 25.0g/L, yeast powder 7.5g/L and NaCl 6.0g/L. And the FB medium without glucose was configured with M9 medium at a special volume proportion to obtain the FB-M9 compound medium.

The mother liquor of M9 medium is 5xM9 and comprises the following components: Na₂HPO₄·7H₂O 64.0g/L, KH₂PO₄ 15.0g/L, NH₄Cl 5.0g/L, NaCl 2.5g/L, MgSO₄ 1.5g/L, CaCl₂ 0.05g/L, and 2% glucose.

A preliminary attempt of the compound medium:
FB-M9: volume ratio between FB:M9 was 7:10, the components are as follows: NaCl 6.7g/L , peptone 25.0g/L, yeast powder 7.5g/L , Na₂HPO4·7H₂O 18.3g/L, KH₂PO₄ 4.3g/L, NH₄Cl 1.4g/L, MgSO₄ 0.4g/L, CaCl₂ 0.01g/L and glucose 0.6g/L.

This invention adopted this composition for bacteria fermentation. The process was as outlined in step 3 of Example 1. The result is shown in table 2, wet bacteria weight obtained from per liter culture solution is significantly higher compared to the one obtained from FB medium. The observed protein production is significantly improved with average production up to 30 mg/L.

**Table 2 Comparison of target protein production from test medium (PMC - SA1/ BL - 21 engineered bacteria)**

| | Fermenting in BF medium | | Fermenting in FB-M9 (7: 10) medium | |
|---|---|---|---|---|
| | Bacterial weight (g). Protein content (mg) | | Bacterial weight (g) | Protein content (mg) |
| 1 | 255.07 | 280.8 | 349.82 | 847.82 |
| 2 | 246.3 | 519.94 | 343.47 | 643.71 |
| 3 | 302.28 | 461.965 | 366 | 779.3 |
| 4 | 276.67 | 465.179 | 388.44 | 946.34 |
| A VG | 270.8 | 431.971 | 361.9325 | 804.2925 |

The final improved FB-M9 medium was obtained by further research and repeated comparison in this invention. The production rate of the target protein can reach 34 mg/L as Table 3 shows, under the same fermentation conditions as in example 1.

The components of the improved FB-M9 medium are as follows: NaCl 6.0g/L, peptone 25.0g/L, yeast powder 7.5g/L, glucose 2.0g/L, Na₂HPO₄·7H₂O 6.8g/L, KH₂PO₄ 3.0g/L, NH₄Cl 1.0g/L, MgSO₄ 0.2g/L and CaCl₂ 0.01g/L. Since methionine was required in the growth of B834 engineered bacterium, in the process of B834 as engineered bacteria methionine (40 mg/L) was added to the final improved FB-M9 medium.

**Table 3. Comparison of productivity between improved FB - M9 medium and other medium**

| | BL-21 | | | B834 | | |
|---|---|---|---|---|---|---|
| Engineered strains and the medium | FB | FB-M9 (7: 10) | Improved FB-M9 | FB | FB-M9 (7: 10) | Improved FB-M9 |
| Average unit production (mg/L) | 10 | 24 | 25 | 22.3 | 30 | 34 |

### Example 3. Optimizing conditions for purifying protein

The basic structure of recombinant polypeptide (PMC- SA1, PMC-SA2, PMC - SA3, PMC-SA4, PMC-SE, PMC-PA, PMC-AM) prepared in this invention is Colicin Ia. The isoelectric point of colicin Ia is about 9.15, therefore the classic purification used is Ion Exchange Chromatography (Qiu XQ et al. An engineered multidomain bactericidal peptide as a model for targeted antibiotics against specific bacteria. Nat Biotechnol, 2003; 21(12): 1480-1485).

The principle is: In a pH 9.0 boric acid buffer system, the majority of PMC-SA molecules exist as positively charged ions. When the CM gel particles with negative charge go through the chromatographic column, the recombinant protein molecules with positive charge was mounted on the CM gel particles due to the electric charge attraction, while the other miscellaneous proteins were washed out of the gel column.

In this example, the other steps were as outlined in example 1, but after the miscellaneous proteins were completely washed out, the elution step was performed using a gradient of boric acid buffer from 0.1 to 0.3M NaCl.

Due to the stronger positive property of Na⁺ ions, the recombinant protein was eluted from CM gel particles by Na⁺ ions. There are two variables to be manipulated in the process of ion exchange and purification for a better protein yield: ① NaCl with different concentrations within 0.05-1M can be chosen respectively to elute protein molecules with different positive charges mounted on CM gel particles ②The amount of CM gel particles used can be optimized: Under conditions with certain ionic strength, the amount of protein carried by CM gel particles is relatively constant. In order to increase the amount of protein carried by gel column, the volume of the gel column must be increased.

CM Sepharose Fast Flow is an anion exchange column gel produced by GE company. According to the manual, every 100 ml gel can bind 9mM of cation. The actual usable binding capacity varies with the nature of the sample in the process of dynamic combination, and molecular weight is inversely proportional with binding capacity. The standard sample that has equivalent molecular weight with the recombinant peptides manufactured according to the present invention is bovine COHb-(Mr69kD), which has theoretically a dynamic binding capacity of 30mg/ml. Namely, with 100 ml CM Sepharose Fast Flow glue to retrieve recombinant protein molecules, the theoretically highest recovery rate is about 300 mg (0.004mM). But according to its manual operation, the actual dynamic binding capacity of recombinant protein molecules to CM gel particles reached only 3 mg/ml, i.e. just 10% of the theoretical binding capacity.

In the experiment, we found that in the latter half process of washing out the miscellaneous protein, the conductance curve shows a small peak (as shown in figure 1 a). According to this phenomenon, we speculate that when there is a large amount of recombinant protein in the sample, due to the limited capacity of CM gel particles to bind target protein, only a small amount of the recombinant protein molecules can be recovered. The recombinant protein not mounted on the CM gel particles is washed out from the gel column together with miscellaneous protein. As the recombinant protein is positively charged, a short rising peak appears in the conductance curve.

In an optimized example of this invention: in order to reduce the loss of recombinant proteins, we reduced the loading amount of sample to 1/3 of the manual regulation, and increased the volume of gel from 150 ml to 600 ml, namely the protein amount in the supernatant fluid: gel particle volume=2.5mg/ml. The loss of the recombinant protein decreased in the process of elution. The experimental data showed that the recovery rate of recombinant proteins was increased 3.5 times; the results are shown in figure 1b.

In addition, we set the gradient concentration of NaCl as 0.1M-0.2M-0.3 M in the boric acid buffer used in the elution step, and 0.2M showed the highest elution efficiency and protein purity, as shown in figure 2b.

### Example 4. Determination of protein purity and activity

### Step 1. SDS-PAGE electrophoresis

The fusion protein samples obtained by optimized conditions of example 4 were analyzed by SDS-PAGE electrophoresis and silver nitrate staining. As shown in figure 2, there is a clear protein band at the point of about 70kD relative molecular weight, namely PMC - SA1 manufactured in this invention in the electrophoresis map (a), the map (b) shows that the protein has escaped the mixed zone through the improved gradient elution in example 4 and the purity is improved. The remaining six kinds of recombinant proteins manufactured through the optimized method of this invention also show similar improved purification.

### Step 2. Determination of the antibacterial activity

With the recombinant protein PMC - SA1 and PMC-AM produced by the improved manufacturing method according to examples 1, 2 and 3, we performed the following antibacterial activity test.

10 ml BM medium supplemented with antimicrobial agents was inoculated with 10µL *Methicillin-resistant staphylococcus aureus* (MRSA, ATCC BAA-42) bacteria liquid (10⁵cfu/ml). According to the antimicrobial agents we set six parallel groups: ampicillin sodium 2ug/ml, oxazocilline 4ug/ml, wild type colicin Ia, PMC-SA and Ph-NM (4ug/ml), and blank control group. Culturing at 37°C, 210rpm, and testing optical density value per hour (595 nm), drawing the bacteriostasis curve, as shown in figure 3.

The bacteriostatic curve shows that the recombinant proteins produced by improved the methods of the present invention have good antibacterial activity.

## Claims

1. A method for highly expressing a recombinant protein from engineered bacteria, wherein the end with hydrophilicity of the recombinant protein is a colicin polypeptide and the other end with hydrophobic nature is a targetting moiety which is capable of binding a
target, the method comprising:
(1) transfecting a recombinant plasmid for the expression of the recombinant protein into E.coli engineered with a pET system to obtain positive monoclonal colonies,
(2) producing seed bacteria solution of the positive monoclonal colonies, and inducing protein expression and large-scale culturing of the seed bacteria solution; the supernatant of the large-scale cultured solution containing expressed recombinant protein,
(3) extracting and purifying the recombinant protein from the supernatant, wherein the *E.coli* engineered bacteria with pET system is E.coli B834 (DE3), wherein the
large-scale culturing medium used for said large-scale culturing of the seed bacteria solution has water as solvent and comprises following components : NaCl 6.0∼6.7g/L, peptone 25.0g/L, yeast powder 7.5g/L, glucose 0.6∼2.0g/L, Na₂HPO₄·7H₂O 6.8∼18.3g/L, KH₂PO₄ 3.0∼4.3g/L, NH₄Cl 1.0∼1.4g/L, MgSO₄ 0.2∼0.4g/L, CaCl₂0.01g/L, methionine 0∼40mg/L.

2. The method according to claim 1, the large-scale culturing medium has water as solvent and comprises following components: NaCl 6.0g/L, peptone 25.0g/L, yeast powder 7.5g/L, glucose 2.0g/L, Na₂HPO₄·7H₂O 6.8g/L, KH₂PO₄ 3.0g/L, NH₄Cl 1.0g/L, MgSO₄ 0.2g/L, CaCl₂0.01g/L, methionine 0∼40mg/L.

3. The method according to claim 1 or 2, wherein said large-scale culturing of the seed bacteria solution comprises following steps: the seed bacteria liquid was added into a container and started growth for 2 to 3 hours at 30°C, when the OD value reached 0.4-0.6, the seed bacteria solution was conducted heat shock at 42°C for 30 minutes, and then cooled down to 37°C and kept growing for 1.5 to 2 hours before being collected.

4. The method according to claim 3, in conducting heat shocks, the IPTG with final concentration 0.5mmol/L was added into said large-scale culturing medium.

5. The method according to claim 1, wherein said extraction and purification of the recombinant protein from the supernatant is performed by CM ion exchange column, and the loading quantity of the supernatant samples depends on the ratio value which is 2.5mg/ml between the weight of the recombinant protein in the supernatant and the volume of Gel particles used in the CM ion exchange column.

6. The method according to claim 5, the elution buffer used for said extraction and purification by CM ion exchange column is boric acid buffer solution with 0.2mol/L NaCl.

7. The method according to claim 1, the recombinant plasmid for the expression of the recombinant protein is selected from the group consisting of pBHC-SA1, pBHC-SA2, pBHC-SA3 pBHC-SA4, pBHC-SE, pBHC-PA and pBHC-PorA1.

8. The use of any method according to claims 1-7 for preparing the recombinant peptides PMC-SA1, PMC-SA2, PMC-SA3, PMC-SA4, PMC-SE, PMC-PA or PMC-AM.

9. A medium used for *E.coli* engineered with a pET system, the medium has water as solvent and comprises following components : NaCl 6.0∼6.7g/L, peptone 25.0g/L, yeast powder 7.5g/L, glucose 0.6∼2.0g/L, Na₂HPO₄·7H₂O 6.8∼18.3g/L, KH₂PO₄3.0∼4.3g/L, NH₄Cl 1.0∼1.4g/L, MgSO₄0.2∼0.4g/L, CaCl₂0.01g/L, methionine 0∼40mg/L.

10. The medium according to claim 9, wherein said *E.coli* engineered bacteria with pET system is *E.coli* B834 (DE3), and the medium has water as solvent and comprises following components: NaCl 6.0g/L, peptone 25.0g/L, yeast powder 7.5g/L, glucose 2.0g/L, Na₂HPO₄·7H₂O 6.8g/L, KH₂PO₄ 3.0g/L, NH₄Cl 1.0g/L, MgSO₄0.2g/L, CaCl₂ 0.01g/L, methionine 0∼40mg/L.

## Patentansprüche

1. Verfahren zum hohen Exprimieren eines rekombinanten Proteins aus gentechnisch veränderten Bakterien, wobei es sich bei dem Ende des rekombinanten Proteins mit Hydrophilie um ein Colicin-Polypeptid handelt und es sich bei dem anderen Ende mit hydrophober Beschaffenheit um eine Targeting-Einheit handelt, die fähig ist, ein Target zu binden, wobei das Verfahren umfasst:
(1) Transfizieren eines rekombinanten Plasmids zur Expression des rekombinanten Proteins in gentechnisch veränderte *E. coli* mit einem pET-System, um positive monoklonale Kolonien zu erhalten,
(2) Herstellen einer Impfbakterien-Lösung der positiven monoklonalen Kolonien und Induzieren der Proteinexpression und des großmaßstäblichen Züchtens der Impfbakterien-Lösung; wobei der Überstand der großmaßstäblich gezüchteten Lösung exprimiertes rekombinantes Protein enthält,
(3) Extrahieren und Reinigen des rekombinanten Proteins aus dem Überstand, wobei es sich bei den gentechnisch veränderten *E. coli* Bakterien mit dem pET-System um *E. coli* B834 (DE3) handelt,
wobei das für das großmaßstäbliche Züchten der Impfbakterien-Lösung verwendete Medium zum großmaßstäblichen Züchten Wasser als Lösungsmittel aufweist und folgende Bestandteile umfasst: NaCl 6,0-6,7 g/l; Pepton 25,0 g/l; Hefepulver 7,5 g/l, Glucose 0,6-2,0 g/l, Na₂HPO₄·7H₂O 6,8-18,3 g/l, KH₂PO₄ 3,0-4,3 g/l, NH₄Cl 1,0-1,4 g/l, MgSO₄ 0,2-0,4 g/l, CaCl₂ 0,01 g/l, Methionin 0-40 mg/l.

2. Verfahren gemäß Anspruch 1, wobei das Medium zum großmaßstäblichen Züchten Wasser als Lösungsmittel aufweist und folgende Bestandteile umfasst: NaCl 6,0 g/l; Pepton 25,0 g/l; Hefepulver 7,5 g/l, Glucose 2,0 g/l, Na₂HPO₄·7H₂O 6,8 g/l, KH₂PO₄ 3,0 g/l, NH₄Cl 1,0 g/l, MgSO₄ 0,2 g/l, CaCl₂ 0,01 g/l, Methionin 0-40 mg/l.

3. Verfahren gemäß Anspruch 1 oder 2, wobei das großmaßstäbliche Züchten der Impfbakterienlösung folgende Schritte umfasst: die Impfbakterienflüssigkeit wurde in einen Behälter hineingegeben und begann das Wachstum 2 bis 3 Stunden lang bei 30°C, als der OD-Wert 0,4-0,6 erreichte, wurde mit der Impfbakterienlösung 30 Minuten lang ein Hitzeschock bei 42°C ausgeführt und sie kühlte dann auf 37°C herunter und wuchs 1,5 bis 2 Stunden lang weiter, bevor sie gewonnen wurde.

4. Verfahren gemäß Anspruch 3, wobei beim Ausführen von Hitzeschocks das IPTG mit einer Endkonzentration von 0,5 mmol/l in das Medium zum großmaßstäblichen Züchten hineingegeben wurde.

5. Verfahren gemäß Anspruch 1, wobei die Extraktion und Reinigung des rekombinanten Proteins aus dem Überstand durch eine CM-Ionenaustauschsäule durchgeführt wird und die Auftragsmenge der Proben des Überstands von dem Verhältniswert abhängt, der 2,5 mg/ml zwischen dem Gewicht des rekombinanten Proteins im Überstand und dem Volumen der in der CM-Ionenaustauschsäule verwendeten Gelpartikel beträgt.

6. Verfahren gemäß Anspruch 5, wobei es sich bei dem für die Extraktion und Reinigung durch CM-Ionenaustauschsäule verwendeten Elutionspuffer um Borsäure-Pufferlösung mit 0,2 mol/l NaCl handelt.

7. Verfahren gemäß Anspruch 1, wobei das rekombinante Plasmid zur Expression des rekombinanten Proteins aus der Gruppe ausgewählt ist, bestehend aus pBHC-SA1, pBHC-SA2, pBHC-SA3, pBHC-SA4, pBHC-SE, pBHC-PA und pBHC-PorA1.

8. Verwendung eines Verfahrens gemäß den Ansprüchen 1-7 zum Herstellen der rekombinanten Peptide PMC-SA1, PMC-SA2, PMC-SA3, PMC-SA4, PMC-SE, PMC-PA oder PMC-AM.

9. Medium, verwendet für gentechnisch veränderte *E. coli* mit einem pET-System, wobei das Medium Wasser als Lösungsmittel aufweist und folgende Bestandteile umfasst: NaCl 6,0-6,7 g/l; Pepton 25,0 g/l; Hefepulver 7,5 g/l, Glucose 0,6-2,0 g/l, Na₂HPO₄·7H₂O 6,8-18,3 g/l, KH₂PO₄ 3,0-4,3 g/l, NH₄Cl 1,0-1,4 g/l, MgSO₄ 0,2-0,4 g/l, CaCl₂ 0,01 g/l, Methionin 0-40 mg/l.

10. Medium gemäß Anspruch 9, wobei es sich bei den gentechnisch veränderten *E. coli*-Bakterien mit dem pET-System um *E. coli* B834 (DE3) handelt und das Medium Wasser als Lösungsmittel aufweist und folgende Bestandteile umfasst: NaCl 6,0 g/l; Pepton 25,0 g/l; Hefepulver 7,5 g/l, Glucose 2,0 g/l, Na₂HPO₄·7H₂O 6,8 g/l, KH₂PO₄ 3,0 g/l, NH₄Cl 1,0 g/l, MgSO₄ 0,2 g/l, CaCl₂ 0,01 g/l, Methionin 0-40 mg/l.

## Revendications

1. Procédé pour l'expression massive d'une protéine recombinante à partir de bactéries modifiées, où l'extrémité de nature hydrophile de la protéine recombinante est un polypeptide de colicine et l'autre extrémité de nature hydrophobe est un fragment de ciblage qui est capable de se lier à une cible, le procédé comprenant :
(1) la transfection d'un plasmide recombinant pour l'expression de la protéine recombinante dans des *E*. *coli* modifiées avec un système pET afin d'obtenir des colonies monoclonales positives,
(2) la production d'une solution de bactéries d'ensemencement des colonies monoclonales positives, et l'induction de l'expression protéique et la culture à grande échelle de la solution de bactéries d'ensemencement ; le surnageant de la solution cultivée à grande échelle contenant la protéine recombinante exprimée,
(3) l'extraction et la purification de la protéine recombinante à partir du surnageant, les bactéries *E*. *coli* modifiées avec le système pET étant *E*. *coli* B834 (DE3),
où le milieu de culture à grande échelle utilisé pour ladite culture à grande échelle de la solution de bactéries d'ensemencement emploie de l'eau comme solvant et comprend les composants suivants : NaCl de 6,0 à 6,7 g/L, peptone 25,0 g/L, poudre de levure 7,5 g/L, glucose de 0,6 à 2,0 g/L, Na₂HPO₄·7H₂O de 6,8 à 18,3 g/L, KH₂PO₄ de 3,0 à 4,3 g/L, NH₄Cl de 1,0 à 1,4 g/L, MgSO₄ de 0,2 à 0,4 g/L, CaCl₂ 0,01 g/L, méthionine de 0 à 40 mg/L.

2. Procédé selon la revendication 1, le milieu de culture à grande échelle employant l'eau comme solvant et comprenant les composants suivants : NaCl 6,0 g/L, peptone 25,0 g/L, poudre de levure 7,5 g/L, glucose 2,0 g/L, Na₂HPO₄·7H₂O 6,8 g/L, KH₂PO₄ 3,0 g/L, NH₄Cl 1,0 g/L, MgSO₄ 0,2 g/L, CaCl₂ 0,01 g/L, méthionine de 0 à 40 mg/L.

3. Procédé selon la revendication 1 ou 2, dans lequel ladite culture à grande échelle de la solution de bactéries d'ensemencement comprend les étapes suivantes : le liquide de bactéries d'ensemencement a été ajouté dans un récipient et on a commencé la croissance pendant 2 à 3 h à 30 °C, lorsque la valeur de DO a atteint 0,4 à 0,6, la solution de bactéries d'ensemencement a été soumise à un choc thermique à 42 °C pendant 30 minutes, puis elle a été refroidie à 37 °C et a continué de croître pendant 1,5 à 2 h avant d'être récoltée.

4. Procédé selon la revendication 3, où pendant la réalisation des chocs thermiques, de l'IPTG avec une concentration finale de 0,5 mmol/L a été ajouté dans ledit milieu de culture à grande échelle.

5. Procédé selon la revendication 1, dans lequel lesdites extraction et purification de la protéine recombinante sont effectuées sur une colonne d'échange ionique CM, et la quantité des échantillons de surnageant introduite dépend de la valeur de rapport qui est de 2,5 mg/ml entre le poids de la protéine recombinante dans le surnageant et le volume de particules de gel utilisées dans la colonne d'échange ionique CM.

6. Procédé selon la revendication 5, le tampon d'élution utilisé pour lesdites extraction et purification sur colonne d'échange ionique CM étant une solution tampon d'acide borique avec 0,2 mol/L de NaCl.

7. Procédé selon la revendication 1, le plasmide recombinant pour l'expression de la protéine recombinante étant choisi dans le groupe constitué de pBHC-SA1, pBHC-SA2, pBHC-SA3 pBHC-SA4, pBHC-SE, pBHC-PA et pBHC-PorA1.

8. Utilisation d'un quelconque procédé selon les revendications 1 à 7 pour préparer les peptides recombinants PMC-SA1, PMC-SA2, PMC-SA3, PMC-SA4, PMC-SE, PMC-PA ou PMC-AM.

9. Milieu utilisé pour *E*. *coli* modifiées avec un système pET, le milieu employant l'eau comme solvant et comprenant les composants suivants : NaCl de 6,0 à 6,7 g/L, peptone 25,0 g/L, poudre de levure 7,5 g/L, glucose de 0,6 à 2,0 g/L, Na₂HPO₄·7H₂O de 6,8 à 18,3 g/L, KH₂PO₄ de 3,0 à 4,3 g/L, NH₄Cl de 1,0 à 1,4 g/L, MgSO₄ de 0,2 à 0,4 g/L, CaCl₂ 0,01 g/L, méthionine de 0 à 40 mg/L.

10. Milieu selon la revendication 9, où lesdites bactéries *E*. *coli* modifiées avec le système pET sont *E. coli* B834 (DE3), et le milieu emploie l'eau comme solvant et comprend les composants suivants : NaCl 6,0 g/L, peptone 25,0 g/L, poudre de levure 7,5 g/L, glucose 2,0 g/L, Na₂HPO₄·7H₂O 6,8 g/L, KH₂PO₄ 3,0 g/L, NH₄Cl 1,0 g/L, MgSO₄ 0,2 g/L, CaCl₂ 0,01 g/L, méthionine de 0 à 40 mg/L.
